(19) Europäisches Patentamt
European Patent Office
Office européen des brevets

(11) EP 0 448 454 B1

(12) FASCICULE DE BREVET EUROPEEN

(45) Date de publication et mention de la délivrance du brevet: **10.01.1996 Bulletin 1996/02**

(51) Int Cl.[6]: **C12P 7/26**, C12P 7/24, C12P 19/02

(21) Numéro de dépôt: **91400725.7**

(22) Date de dépôt: **18.03.1991**

# (54) Procédé enzymatique de préparation de désoxycétoses

Enzymatisches Verfahren zur Herstellung von Desoxyketosen

Enzymatic process for the preparation of desoxyketoses

(84) Etats contractants désignés:
**CH DE FR LI NL**

(30) Priorité: **19.03.1990 FR 9003469**

(43) Date de publication de la demande:
**25.09.1991 Bulletin 1991/39**

(73) Titulaire: **SYSTEMS BIO-INDUSTRIES**
**F-75008 Paris (FR)**

(72) Inventeurs:
- **Delest, Philippe**
  **F-78590 Noisy-le-Roi (FR)**
- **Demuynck, Colette**
  **F-63450 St Amant-Tallende (FR)**

(74) Mandataire: **Polus, Camille et al**
**F-75441 Paris Cedex 09 (FR)**

(56) Documents cités:
- **TETRAHEDRON LETTERS, vol. 28, no. 45, 1987, Pergamon Journals, GB; J. BOLTE et al., pp. 5525-5528/**
- **CHEMICAL ABSTRACTS, vol. 112, no. 11, 12 mars 1990, Columbus, OH (US); Z. LIANG, p. 461, no. 95532t/**
- **CHEMICAL ABSTRACTS, vol. 104, no. 13, 31 mars 1986, Columbus, OH (US); C. YU et al., p. 271, no. 104540h/**
- **CHEMICAL ABSTRACTS, vol. 104 no. 13, 31 mars 1986, Columbus, OH (US); N.J. WALTON et al., p. 430, no. 106363b/**
- **CHEMICAL ABSTRACTS, vol. 82, no. 11, 17 mars 1975, Columbus, OH (US); A.I. CARPE et al., p. 164, no. 69722x/**
- **CHEMICAL ABSTRACTS, vol. 101, no. 9, 27 août 1984, Columbus, OH (US); A. YOKOTA et al., p. 346, no. 69109p/**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1)Convention sur le brevet européen).

EP 0 448 454 B1

**Description**

La présente invention concerne la préparation enzymatique de désoxycétoses.

Les désoxycétoses, qui répondent à la formule

$$CH_3(CHOH)_nCOCH_2OH$$

dans laquelle n vaut 1 à 4, sont peu abondants dans la nature.

L'industrie alimentaire a besoin d'arômes "naturels" pour les incorporer dans divers produits alimentaires, un grand nombre de consommateurs ne voulant plus d'additifs artificiels. Aussi, la mise au point de procédés d'obtention d'arômes que l'on peut qualifier de naturels est nécessaire; de tels procédés ne doivent mettre en oeuvre que des matières premières naturelles, ce qui exclut l'utilisation des réactifs habituels de la chimie organique. L'industrie s'est ainsi tournée vers la réalisation de réactions enzymatiques mettant en jeu des microorganismes ou des enzymes, notamment d'origine végétale. La présente invention permet de préparer des produits naturels qui sont des composants d'arômes ou qui pourront ensuite être transformés en ceux-ci.

Ainsi, par exemple, on peut préparer par ce procédé à partir de produits naturels par voie enzymatique, le désoxy-6 L-sorbose, l'un des stéréoisomères du désoxy-6 hexulose-2, qui est un précurseur du Furanéol®, composant de l'arôme caramel, de formule

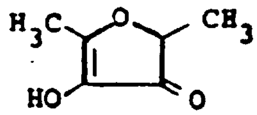

comme décrit dans J. Org. Chem. 48 p. 3493-3497 (1983).

L'obtention de cétoses de formule

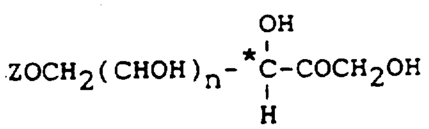

dans laquelle Z représente H ou $PO_3H_2$ et n un nombre entier de 0 à 3, par action d'un aldéhyde de formule

$$ZOCH_2\text{-}(CHOH)_{n-1}\text{-}CHO$$

sur l'acide hydroxypyruvique en présence d'une transcétolase a été décrite dans Tetrahedron Letters 28 (45) p. 5525-5528 (1987).

Par ailleurs, lors d'études du métabolisme végétal, reportées notamment dans les articles résumés dans Chem. Abs. 82 69722 x, 104 104540 h, 104 106363 b et 112 95532 t, la présence d'aminotransférases dans les plantes a été mise en évidence ainsi que leur intervention dans une réaction de transamination entre des aminoacides, notamment la sérine, la glycine et l'alanine, et des alphacétoacides, notamment les acides glyoxylique et pyruvique, réaction réversible qui conduit notamment à la formation d'hydroxypyruvate et de glycérate.

La Demanderesse a maintenant constaté que les substrats de la transcétolase ne sont pas uniquement des aldéhydes porteurs d'un groupe hydroxyle sur chaque carbone de la chaîne, mais que l'on peut obtenir par réaction de l'acide hydroxypyruvique avec un aldéhyde de formule $CH_3(CHOH)_nCHO$, des cétoses, avec un faible rendement lorsque n = O; par contre, lorsque l'acide hydroxypyruvique est généré in situ, par action de L-sérine et d'acide glyoxylique en présence de glyoxylate transaminase, le rendement de la réaction est supérieur, et le rendement global est nettement supérieur à celui des deux réactions effectuées successivement.

La présente invention a donc, selon un premier aspect, pour objet un procédé de préparation d'un désoxycétose, composé de formule

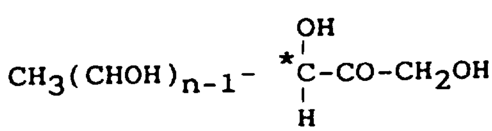

I

dans laquelle n est un entier de 1 à 4 et le carbone marqué d'un astérisque est en configuration S, caractérisé en ce que l'on fait réagir la L-sérine, l'acide glyoxylique et un aldéhyde de formule $CH_3(CHOH)_{n-1}CHO$ en présence d'une glyoxylate aminotransférase, d'une transcétolase et de thiamine pyrophosphate.

Le principe de ce procédé est résumé par le schéma réactionnel suivant :

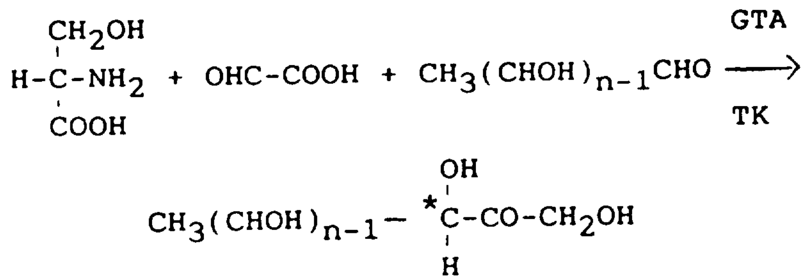


dans lequel GTA représente une glyoxylate aminotransférase, ou glyoxylate transaminase, et TK une transcétolase.

La réaction d'aldolisation est stéréospécifique et stéréosélective; lorsque les aldéhydes comportent un carbone asymétrique en alpha du carbonyle, seul l'isomère R est transformé.

La L-sérine peut être obtenue par hydrolyse de diverses protéines ou par fermentation selon des procédés connus; l'acide glyoxylique se trouve dans les fruits et légumes non matures ou peut être préparé par fermentation; ces deux produits de départ peuvent donc être "naturels".

La glyoxylate transaminase se trouve dans tous les végétaux, et par exemple dans les feuilles d'avoine comme décrit dans Biochim. Biophys. Acta 321, p. 156-164 (1973), dans les haricots comme décrit dans Can. J. Biochem. 48, p. 486-492 (1970) ou dans les peroxisomes d'épinard comme décrit dans J. Biol. Chem 245, 3821-3830 (1970).

La transcétolase est présente dans de nombreux végétaux et dans des levures comme la levure de boulanger; la société Sigma commercialise une transcétolase de *Saccharomyces cerevisiae*.

Selon un aspect préféré de l'invention, les enzymes sont extraites des feuilles d'épinard, où elles sont présentes toutes les deux en quantité importante.

La réaction est effectuée en milieu aqueux, de préférence tamponné à un pH compris entre 7 et 8, en présence d'un coenzyme de la transcétolase, tel que la thiamine pyrophosphate, et éventuellement de préférence de phosphate de pyridoxal et du cation $Mg^{++}$.

Comme dans tout procédé enzymatique de ce type, la concentration des réactifs et des enzymes est déterminée par des essais préalables. En général la concentration des enzymes, mesurée en activité vis-à-vis de leur substrat naturel, sera de l'ordre de 2 à 6 unités par ml, tandis que celle de la L-sérine, de l'acide glyoxylique ou de l'aldéhyde sera de 0,1 M à 0,3 M. La concentration en thiamine pyrophosphate sera comprise entre 0,1 µM et 2 mM, tandis que le phosphate de pyridoxal pourra être introduit à une concentration de 3 µM à 2 mM, et le $Mg^{++}$ de 0,2 mM à 8 mM.

Les produits de départ, la L-sérine, l'acide glyoxylique, et l'aldéhyde peuvent être introduits, dès le début de la réaction, en quantités équimoléculaires; néanmoins, l'acide glyoxylique a un certain effet inhibiteur sur les réactions et on préfère l'ajouter par fractions successives ou en continu, dans le milieu réactionnel; de préférence, la concentration de cet acide ne dépassera pas 20 mM.

Le procédé selon l'invention, peut être mis en oeuvre avec des aldéhydes de synthèse ou préexistant dans la nature ou encore obtenues par des procédés de type biologique, par hydrolyse ou réaction enzymatique.

Ainsi l'acétaldéhyde est obtenu par oxydation enzymatique de l'éthanol.

Le dihydroxy-2,3 butyraldéhyde n'existe pas dans la nature et un autre objet de l'invention est le procédé d'obtention par voie enzymatique, à partir de produits qui peuvent être trouvés dans la nature, du composé de formule II

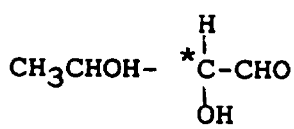

mélange des 2 diastéréoisomères, dans lesquels le carbone, marqué d'un astérisque, en alpha du carbonyle est en configuration R.

Selon la configuration du carbone en β de l'aldéhyde, la formule II représente le désoxy-4 L-thréose ou le désoxy-4 D-érythrose; ces composés sont des produits utilisés dans la préparation selon le procédé de la présente invention, respectivement du désoxy-6 L-sorbose et du désoxy-6 D-fructose.

Le procédé de préparation de l'aldéhyde de formule II consiste à soumettre le composé de formule III

$$CH_3CHOHCOCH_2OH$$

à l'action d'une polyol déshydrogénase (PDH) puis le triol obtenu à l'action d'une xylose réductase (XR), selon le schéma réactionnel

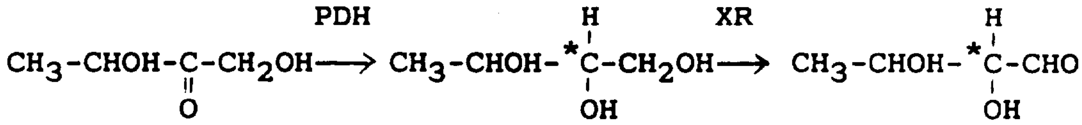

III II

La première réaction est stéréospécifique, et le carbone asymétrique apparu est en configuration R.

Lorsque le composé de formule III est préparé par le procédé de l'invention, à partir de L-sérine, d'acide glyoxylique et d'acétaldéhyde en présence de GTA et TK, il se présente sous forme d'un seul stéréoisomère, le désoxy-4L-érythrulose, et le composé II obtenu est le désoxy-4 L-thréose, de formule

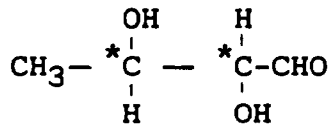

Les 2 étapes du procédé sont avantageusement réalisées simultanément, introduisant dans le milieu réactionnel soit un mélange des 2 enzymes isolées soit un microorganisme dans lequel les 2 enzymes sont présentes.

Lorsque les 2 étapes sont effectuées successivement, on doit introduire dans le milieu réactionnel du NADH pour la première étape et du NAD pour la seconde, avec un système pour régénérer le cofacteur; lorsque les 2 étapes sont effectuées simultanément, il suffit d'introduire dans le milieu une quantité suffisante de NADH; enfin, en présence d'un microorganisme, l'introduction de l'un ou l'autre de ces cofacteurs n'est pas nécessaire.

Les enzymes sont connues.

Parmi les polyols déshydrogénases convenant, on peut citer la polyol déshydrogénase commercialisée par Sigma, extraite de *Candida utilis* ou la sorbitol déshydrogénase extraite de foie de mouton, commercialisée par Sigma et la glycérol déhydrogénase.

Cette dernière peut être extraite de *Bacillus megaterium, Cellulomonas species, Enterobacter aerogenes, Aspergillus niger*, elle est aussi commercialisée par la société Sigma.

On sait qu'une xylose réductase peut être extraite *d'Enterobacter liquefaciens* (ou *Serratia liquefaciens*) comme décrit dans Agr. Biol. Chem. 40(8) p. 1493-1503 (1976) ou de *Corynebacterium species* comme décrit dans Agr. Biol. Chem. 40(8) p. 1485-1491 (1976).

On a constaté que les deux enzymes sont présentes dans diverses bactéries, notamment dans *Serratia liquefaciens* et *Corynebacterium equi*, ou dans des levures notamment *Rhodotorula glutinis*, *Candida utilis* ou *albicans* ou encore dans des champignons, notamment des *Aspergillus* ou des *Penicillium*, et on préfère effectuer simultanément les deux étapes du procédé, en mettant le composé de formule III racémique ou l'un de ses énantiomères en présence des microorganismes entiers ou des extraits enzymatiques de ceux-ci préparés de façon classique, par rupture mécanique des parois cellulaires par broyage et sonication ou par hydrolyse enzymatique comme décrit dans Current Microbiology 8, p.383-88 (1982) et dans J. General Microbiology 135, p.1391-1394 (1989).

Les réactions sont effectuées en milieu aqueux, de préférence à un pH compris entre 7 et 8 pour la première étape, à un pH compris entre 8,5 et 10 pour la seconde étape ou lorsque les 2 étapes sont simultanées.

La présence dans le milieu réactionnel de composés connus pour améliorer l'efficacité des enzymes est souhaitée et notamment celle, pour la xylose réductase, du cation $Mg^{++}$ à une concentration comprise entre 0,5 mM et 10 mM. Les concentrations des produits de départ et des enzymes sont déterminées par des essais préalables. Elles seront par exemple de l'ordre de 0,05 M à 0,3 M pour les produits et de 2 à 6 unités/ml pour les enzymes.

Selon une variante de l'invention l'aldéhyde utilisé dans le procédé de préparation par voie enzymatique, à partir de produits d'origine naturelle du désoxy-6 L-sorbose, a pour formule développée

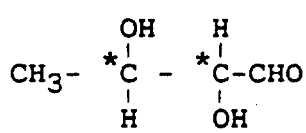

L'aldéhyde peut être préparé par action sur la cétone correspondante

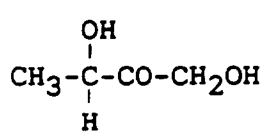

obtenue par réaction de la L-sérine, de l'acide glyoxylique et d'acétaldéhyde naturels en présence d'une glyoxylate aminotransférase et de transcétolase, d'une polyol déshydrogénase et d'une xylose réductase.

Dans ce qui suit, on décrit des exemples de mise en oeuvre du procédé de l'invention appliqué à la préparation du désoxy-4 L-érythrulose à partir de l'acétaldéhyde, du désoxy-6 hexulose-2 à partir du dihydroxy-2,3 butyraldéhyde racémique, du désoxy-6 L-sorbose par voie naturelle, ainsi qu'un exemple du procédé enzymatique de préparation du composé de formule II. Une méthode d'obtention des deux enzymes : glyoxylate transaminase et transcétolase, à partir des feuilles d'épinard et une méthode d'extraction des enzymes de *Serratia liquefaciens* et *Corynebacterium equi* sont mentionnés.

Obtention des enzymes de l'épinard

L'extraction, la purification et les centrifugations sont effectuées à 0°C. Les centrifugations sont à 10000 g.

A) Extrait de transcétolase :

300 g de feuilles fraîches d'épinard sont broyées finement en présence de 500 ml de tampon phosphate 0,01 M de $K_2HPO_4$, dont le pH est 9. Les débris végétaux sont éliminés par une centrifugation suivie d'une filtration. On introduit ensuite dans le filtrat 89,5 g de $(NH_4)_2SO_4$, et on élimine le précipité formé par centrifugation. Après addition de 70 g de $(NH_4)_2SO_4$ dans le surnageant, séparé du culot de centrifugation, on centrifuge la suspension formée. Le culot est isolé et servira à préparer la glyoxylate aminotransférase. Le surnageant est traité de nouveau par 36 g de $(NH_4)_2SO_4$ et on centrifuge de nouveau. Le culot de centrifugation contient la transcétolase. Il est mis en suspension dans 10 ml d'eau distillée, puis chromatographié sur une colonne de Sephadex ® G75 de 2,5 cm de diamètre et 30 cm de hauteur, en éluant avec de l'eau distillée. On recueille l'éluat par fractions de 20 ml; la quantité de transcétolase contenue dans chacune d'elles est déterminée comme suit.

On introduit, à 25°C, 20 µl d'éluat contenant la transcétolase dans 1 ml d'une solution aqueuse tamponnée à pH 7,5 par de la glycylglycine 0,1 M et contenant du D-xylulose-5 phosphate (9 mM), du D-ribose-5 phosphate (15 mM) du pyrophosphate de thiamine (1,5 mM), du $MgCl_2$ (8 mM) et du NADH (0,42 mM) ainsi qu'une unité de triosephosphateisomérase et 10 unités de glycérophosphate déshydrogénase, commercialisées par Sigma; une unité triosephosphateisomerase convertit 1 pmole de D-glycéraldéhyde-3 phosphate, à pH 7,6 et 25°C, en 1 minute, tandis qu'une unité de glycérophoshate déshydrogénase convertit 1 pmole de dihydroxyacétone phosphate, à pH 7,4 et 25°C, en 1 minute.

La densité optique de l'échantillon est mesurée à 340 nm toutes les minutes; elle est proportionnelle à la concentration de NADH présente. On détermine alors la pente sDO de la courbe représentant l'évolution de la densité optique en fonction du temps et on calcule le nombre d'unités par ml de l'extrait (U/ml) en appliquant l'équation suivante :

$$U/ml = \frac{sDO \times V\ échantillon}{6,22 \times V\ éluat}$$

B) Extrait de glyoxylate aminotransférase

Le culot isolé précédemment est lavé avec 400 ml d'acétone à -40°C. On obtient ainsi une poudre qui est introduite dans 400 ml d'un tampon phosphate ($K_2HPO_4$,0,01M) de pH 9 et les protéines insolubles sont éliminées par centrifugation. On effectue ensuite trois précipitations successives en introduisant dans les surnageants de centrifugation 50 g de $(NH_4)_2SO_4$. Le dernier culot de centrifugation est isolé et chromatographié sur une colonne de Sephadex ®, comme pour la préparation de la transcétolase.

La quantité de glyoxylate aminotransférase contenue dans chaque fraction de l'éluat est déterminée comme suit.

On introduit, à 25°C, 5 µl d'extrait dans 1 ml de tampon glycylglycine 0,1 M à pH 7,6, contenant de la L-sérine (20 mM), de l'acide glyxoxylique (1,35 mM), du phosphate de pyridoxal (0,37 mM), du NADH (0,42 mM) et 0,1 unité de glyoxylate réductase commercialisée par Sigma; une unité de glyoxylate réductase convertit, à 25°C, 1 pmole d'hydroxypyruvate en D-glycérate par minute.

La densité optique de l'échantillon est mesurée toutes les minutes, à 340 nm et on détermine le nombre d'unités/ml de l'extrait par la même formule qu'en A).

Obtention des extraits enzymatiques bactériens :

A) - de *Serratia liquefaciens* :

5,25 g de cellules de la souche déposée à l'Institut for Fermentation, Osaka (Japon) sous le N° CIP 103328T sont mis en suspension dans 75 ml de tampon TRIS de pH 7,5, contenant 0,05% (p/V) de mercaptoéthanol; le mélange est soumis à une sonication à la température de la glace, pendant 5 minutes à 200 watts puis centrifugé, pendant 20 minutes

à 4°C, à la vitesse de 11000 tours par minute. Le surnageant est isolé.

B) - de *Corynebacterium equi* :

On introduit dans 100 ml de culture de cellules de la souche déposée à la Collection de Microorganismes de l'Institut Pasteur, Paris (France) sous la référence IFO 3730, en fin de phase exponentielle, $10^6$ unités de penicilline G pour fragiliser les parois cellulaires. Le milieu centrifugé au bout de 4 heures et le culot introduit dans 20 ml de tampon TRIS (30 mM, pH = 7,5) avec 300 mg de lysozyme, 50 unités de mutanolysine et de l'acide éthylènediaminetétraacétique (EDTA) à la concentration de 50 mM. Après 12 heures à 37° C, on centrifuge à 11.000 tours/min pendant 20 minutes et isole le surnageant.

Les activités spécifiques des enzymes contenues dans les extraits sont déterminés pour PDH par mesure de la transformation du L-erythrulose (50 mM) en L-thréytol ou de la dihydroxy-1,3 butanone-2 (50 mM) en désoxy-4L-thréose, en présence de NADH (10 mg/ml) dans du tampon TRIS (pH - 7,5), et pour XR par mesure de la transformation de l'erythrytol (100 mM) en L-thréose en présence de NAD (0,2 mM) et $MgCl_2$ (2 mM) dans un tampon de pH 9,7.

Préparation de l'acide hydroxypyruvique (sous forme de sel sodique) :

A 25°C, on introduit dans 20 ml d'extrait enzymatique de glyoxylate aminotransférase, contenant 80 unités enzymatiques, sous agitation, en atmosphère d'azote à l'abri de la lumière, 0,476 g de tampon HEPES, 0,210 g de L-sérine, 0,148 g d'acide glyoxylique et 9,52 mg de pyridoxal-5 phosphate. Le pH du milieu est amené à 7,5 par addition d'une solution aqueuse de NaOH N.

Dans ces conditions, il s'est formé au bout de 5 heures, 55 mg d'hydroxypyruvate, mais si la glyoxylate est introduit par fractions au cours des 24 heures, le rendement est de 65%.

Dans ces essais la concentration en hydroxypyruvate a été déterminée par chromatographie en phase liquide haute performance sur une colonne Polypore ®.

EXEMPLE 1 - Préparation du désoxy-4 L-érythrulose :

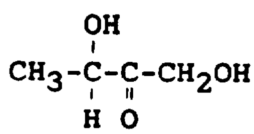

L'acétaldéhyde utilisé dans cette réaction st obtenu par oxydation enzymatique de l'éthanol; on introduit sous agitation, à 25°C, sous atmosphère d'azote, dans 60 ml de tampon HEPES 0,1 M, préparé avec 30 ml d'extrait de transcétolase, contenant 340 unités enzymatiques, et 30 ml d'extrait de glyoxylate aminotransférase, contenant 160 unités enzymatiques, 0,953 g de L-sérine (9 mmole), 0,017 g de $MgCl_2$, 0,055 g de thiamine pyrophosphate et 0,0318 g de phosphate de pyridoxal, puis 0,11 g d'acide glyoxylique et 0,200 g d'acétaldéhyde; au bout de 4 heures, on ajoute de nouveau 0,200 g d'acétaldéhyde et toutes les 4 heures environ, 0,110 g d'acide glyoxylique. Le pH est amené à 7,5 par addition d'une solution aqueuse de NaOH N.

Au bout de 24 heures, on obtient 160 mg de désoxy-4 L-érythrulose, dont le pouvoir rotatoire est:

$$[\alpha]_D^{25} = 4,2 \ (CH_3OH, \ C = 0,059 \ g/l)$$

EXEMPLE 2 - Préparation du désoxy-6 hexulose-2 :

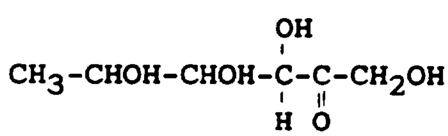

On introduit dans 41 ml de tampon HEPES 0,1 M, à 25°C, sous agitation, 25 ml d'extrait de glyoxylate aminotransférase contenant 82 unités enzymatiques, et 25 ml d'extrait de transcétolase contenant 85 unités enzymatiques, 0,434 g de L-sérine, 0,06 g de glyoxylate de sodium 1,345 g de dihydroxybutyraldéhyde racémique, 0,011 g de $MgCl_2$, 0,038

g de thiamine pyrophosphate et 0,022 g de phosphate de pyridoxal; le pH est amené à 7,5 par addition d'une solution aqueuse de NaOH N; 4 autres fractions de 0,06 g de glyoxylate sont ajoutées à 7-8 heures d'intervalle. Après 48 heures, on introduit 120 ml de méthanol dans le milieu réactionnel et sépare les protéines qui ont précipité par centrifugation à 10000 g pendant 5 minutes. Le surnageant est concentré à sec sous pression réduite à 30°C, et l'huile jaune résiduelle, solubilisée dans 2 ml d'eau, est chromatographiée sur une colonne de Dowex ® 50X, sous forme $H^+$, de 2,5 cm de diamètre et 40 cm de hauteur, en éluant avec de l'eau distillée. Les fractions contenant le désoxy-6 hexulose, mis en évidence par chromatographie en couche mince sur silice en éluant avec un mélange $CHCl_3/CH_3OH$ (8/2-v/v), sont chromatographiées sur une colonne de 15 g de résine Amberlite ®, faiblement basique, ce qui les neutralise.

Le solvant est éliminé de l'éluat par distillation sous pression réduite. On obtient ainsi 378 mg du produit recherché, ce qui correspond à un rendement de 30,7 % par rapport au dihydroxybutyraldéhyde introduit.

Pour caractériser le produit final, on a préparé son diacétate par action de l'anhydride acétique dans la pyridine (10/1;v/v) à 0°C : on introduit 378 mg du produit dans 12 ml de la solution d'anhydride, puis après 24 heures on neutralise par addition d'une solution aqueuse concentrée de $NaHCO_3$, et on extrait par le chlorure de méthylène la phase organique qui, lavée, est diluée avec un volume de toluène et le solvant chloré est éliminé sous pression réduite; le résidu toluénique est chromatographié sur une colonne de silice en éluant avec un mélange d'éther éthylique et de pentane (2/1; v/v). On isole ainsi les tétraacétates du mélange des deux diastéréoisomères du désoxy-6 hexulose-2 attendus, qui sont le désoxy-6 D-fructose et le désoxy-6 L-sorbose; les pouvoirs rotatoires de ces acétates, mesurées à 25°C dans le chloroforme sont respectivement de + 20° (C = 0,039 g/l) et -18° (C = 0,036 g/l).

<u>EXEMPLE 3</u> - Préparation du composé de formule II.

On introduit dans 18 ml de tampon TRIS (0,05 M, pH = 7,5) de la dihydroxy-1,3 butanone-2 (200 mM) et 2 g de cellules de *Corynebacterium liquefaciens* humides, séparées de leur milieu de culture à la fin de la phase exponentielle de croissance.

Après 48 heures à température ambiante, on atteint un état d'équilibre; la concentration en composé de formule II est de 40 mM environ, ce qui correspond à un rendement de 20%.

<u>EXEMPLE 4</u> - Préparation des désoxy-6 hexulose-2 : désozy-6 L-sorbose et désoxy-6 D-fructose.

Le milieu réactionnel contenant le composé de formule II, obtenu à l'exemple 3 est centrifugé et on introduit dans 15 ml de surnageant, 15 ml d'extrait de transcétolase (120 U), puis de l'hydroxypyruvate (5 mM), de la thiamine pyrophosphate (2 mM) et du $MgCl_2$ (3 mM) et on ajuste le pH à 7,5. Après 15 heures à température ambiante, tout l'aldéhyde a été transformé et après traitement du milieu réactionnel comme à l'exemple 2, on obtient les désoxy-6 hexulose-2, à raison de 3 g/l de milieu.

<u>EXEMPLE 5</u> - Préparation du désoxy-6 L-sorbose.

On introduit dans 20 ml de tampon TRIS (0,05 M, pH = 7,5), 120 unités de transcétolase, 2 g de cellules de *Corynebacterium,* du désoxy-4L-érythrulose (100 mM), de l'hydroxypyruvate (100 mM), du $MgCl_2$ (2 mM), de la thiamine pyrophosphate (3 mM). Après 48 heures, sous agitation, à 30°C, on introduit 120 ml de méthanol dans le milieu et on sépare les protéines précipitées par centrifugation à 8000 tours/minute pendant 10 minutes.

Le rendement de la réaction est de 20% après traitement du milieu comme à l'exemple 2. Le rendement est supérieur lorsqu'on introduit un excès d'hydroxypyruvate par additions successives.

## Revendications

1. Procédé de préparation d'un composé de formule

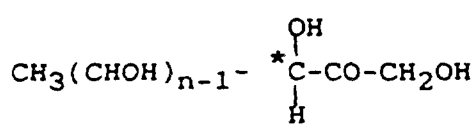

dans laquelle n est un entier de 1 à 4 et le carbone marqué d'un astérisque est en configuration S, caractérisé en ce que l'on fait réagir la L-sérine, l'acide glyoxylique et un aldéhyde de formule $CH_3(CHOH)_{n-1}$-CHO en présence d'une glyoxylate aminotransférase, d'une transcétolase et de thiamine pyrophosphate.

2. Procédé selon la revendication 1, caractérisé en ce que le milieu réactionnel contient du phosphate de pyridoxal

et $Mg^{++}$.

3. Procédé selon l'une des revendications 1 à 2, caractérisé en ce que les enzymes sont extraites des feuilles d'épinard.

4. Procédé selon l'une des revendications 1 à 3, caractérisé en ce que l'acide glyoxylique est introduit par fractions dans le milieu réactionnel.

5. Procédé selon la revendication 1, caractérisé en ce que l'aldéhyde de formule $CH_3$-$(CHOH)_{n-1}$-CHO a pour formule développée

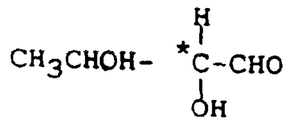

dans laquelle le carbone en alpha de l'aldéhyde est en configuration R, ledit aldéhyde étant préparé par action d'une polyol déshydrogénase sur une cétone de formule

$$CH_3CHOHCOCH_2OH$$

le composé obtenu étant soumis ensuite à l'action d'une xylose réductase.

6. Procédé selon la revendication 5, caractérisé en ce que les deux étapes enzymatiques sont effectuées simultanément, en présence de NADH.

7. Procédé selon la revendication 6 , caractérisé en ce que les enzymes sont celles présentes dans un même microorganisme, bactérie, champignon ou levure.

8. Procédé selon la revendication 7 , caractérisé en ce que le microorganisme est choisi parmi *Serratia liquefaciens* ou *Corynebacterium equi.*

9. Procédé selon la revendication 5, caractérisé en ce que les enzymes sont introduites dans le milieu réactionnel dans leurs microorganismes d'origine choisis parmi *Serratia liquefaciens* et *Corynebacterium equi.*

10. Procédé selon la revendication 9 , caractérisé en ce que les cellules sont celles de *Corynebacterium equi* et que l'on introduit dans le milieu réactionnel du $Mg^{++}$ et de la thiamine pyrophosphate.

11. Procédé selon la revendication 1, caractérisé en ce que l'aldéhyde de formule $CH_3$-$(CHOH)_{n-1}$-CHO a pour formule développée

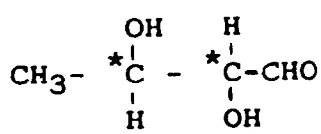

ledit aldéhyde étant préparé selon le procédé de l'une quelconque des revendications 5 à 10, à partir de la cétone de formule

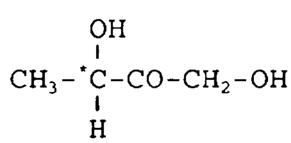

**Patentansprüche**

1. Verfahren zur Herstellung einer Verbindung der Formel

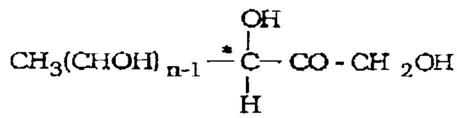

in der n eine ganze Zahl mit einem Wert von 1 bis 4 darstellt und das mit einem Stern gekennzeichnete Kohlenstoffatom in der Konfiguration S vorliegt, **dadurch gekennzeichnet**, daß man L-Serin, Glyoxylsäure und einen Aldehyd der Formel $CH_3$-$(CHOH)_{n-1}$-CHO in Gegenwart einer Glyoxylat-Aminotransferase, einer Transketolase und Thiamin-pyrophosphat umsetzt.

**2.** Verfahren nach Anspruch 1, **dadurch gekennzeichnet**, daß das Reaktionsmedium Pyridoxalphosphat und $Mg^{++}$ enthält.

**3.** Verfahren nach einem der Ansprüche 1 bis 2, **dadurch gekennzeichnet**, daß die Enzyme aus Spiratblättem extrahiert worden sind.

**4.** Verfahren nach einem der Ansprüche 1 bis 3, **dadurch gekennzeichnet**, daß die Glyoxylsäure portionsweise in das Reaktionsmedium eingetragen wird.

**5.** Verfahren nach Anspruch 1, **dadurch gekennzeichnet**, daß der Aldehyd der Formel $CH_3$-$(CHOH)_{n-1}$-CHO der folgenden Strukturformel entspricht

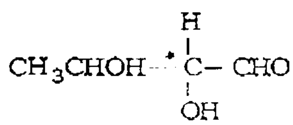

in der das in der alpha-Stellung zu der Aldehydgruppe stehende Kotlenstoffatom in der Konfiguration R vorliegt, welcher Aldehyd durch Einwirken einer Polyol-Deshydrogenase auf ein Keton der Formel

$$CH_3CHOHCOCH_2OH$$

und Behandeln der erhaltenen Verbindung mit einer Xylose-Reduktase hergestellt worden ist.

**6.** Verfahren nach Anspruch 5, **dadurch gekennzeichnet**, daß die beiden enzymatischen Stufen gleichzeitig in Gegenwart von NADH durchgeführt werden.

**7.** Verfahre nach Anspruch 6, **dadurch gekennzeichnet**, daß die Enzyme jene sind, die in dem gleichen Mikroorganismus, Bakterium, Pilz oder der gleichen Hefe vorhanden sind.

**8.** Verfahren nach Anspruch 7, **dadurch gekennzeichnet**, daß der Mikroorganismus aus Serratia liquefaciens oder Corynebacterium equi ausgewählt wird.

**9.** Verfahren nach Anspruch 5, **dadurch gekennzeichnet**, daß die Enzyme in ihren Ausgangsmikroorganismen ausgewahlt aus Serratia liquefaciens und Corynebacterium equi in das Reaktionsmedium eingebracht werden.

**10.** Verfahren nach Anspruch 9, **dadurch gekennzeichnet**, daß die Zellen jene von Corynebacterium equi sind und dem Reaktionsmedium $Mg^{++}$ und Thiaminpyrophosphat zugesetzt wird.

**11.** Verfahren nach Anspruch 1, **dadurch gekennzeichnet**, daß der Aldehyd der Formel $CH_3$-$(CHOH)_{n-1}$-CHO die Strukturformel

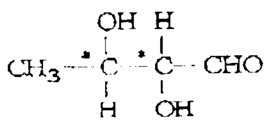

besitzt, welcher Aldehyd nach dem Verfahren gemäß einem der Ansprüche 5 bis 10 ausgehend von dem Keton der Formel

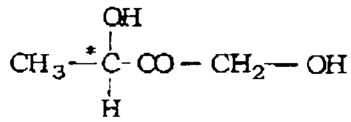

hergestellt worden ist.

## Claims

1. Process for preparing a compound of formula

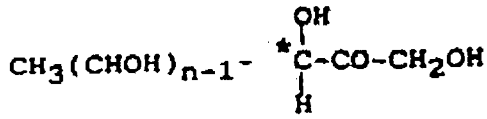

wherein n is an integer from 1 to 4 and the carbon marked with an asterisk is in the S configuration, characterised in that L-serine, glyoxylic acid and an aldehyde of formula $CH_3(CHOH)_{n-1}$-CHO are reacted in the presence of an aminotransferase glyoxylate, a transketolase and thiamine pyrophosphate.

2. Process according to claim 1, characterised in that the reaction medium contains pyridoxal phosphate and $Mg^{++}$.

3. Process according to one of claims 1 and 2, characterised in that the enzymes are extracted from spinach leaves.

4. Process according to one of claims 1 to 3, characterised in that the glyoxylic acid is introduced into the reaction medium in fractions.

5. Process according to claim 1, characterised in that the developed formula of the aldehyde of formula $CH_3(CHOH)_{n-1}$-CHO is:

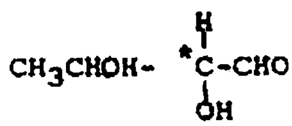

wherein the carbon in the alpha position of the aldehyde is in the R configuration, said aldehyde being prepared by the action of polyol dehydrogenase on a ketone of formula

$$CH_3CHOHCOCH_2OH,$$

the compound obtained then being reacted with a xylose reductase.

6. Process according to claim 5, characterised in that the two enzymatic steps are carried out simultaneously, in the presence of NADH.

7. Process according to claim 6, characterised in that the enzymes are those which are present in one particular microorganism, bacterium, fungus or yeast.

8. Process according to claim 7, characterised in that the microorganism is selected from *Serratia liquefaciens* or *Corynebacterium equi*.

9. Process according to claim 5, characterised in that the enzymes are introduced into the reaction medium in their microorganisms of origin, selected from *Serratia liquefaciens* and *Corynebacterium equi.*

10. Process according to claim 9, characterised in that the cells are those of *Corynebacterium* equi and that $Mg^{++}$ and thiamine pyrophosphate are introduced into the reaction medium.

11. Process according to claim 1, characterised in that the developed formula of the aldehyde of formula $CH_3(CHOH)_{n-1}$-CHO is:

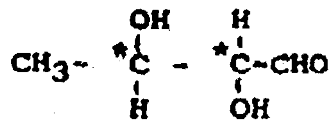

said aldehyde being prepared according to the process in any one of claims 5 to 10, starting from the ketone of formula